# EUROPEAN PATENT SPECIFICATION

(11) **EP 2 066 638 B1**
(45) Date of publication and mention of the grant of the patent: **23.02.2011**
(21) Application number: 07788278.5
(22) Date of filing: 07.08.2007
(51) Int. Cl.: C07D 215/18, A61K 31/4704

(54) **PROCESS FOR THE PURIFICATION OF MONTELUKAST**
VERFAHREN ZUR AUFREINIGUNG VON MONTELUKAST
PROCÉDÉ DE PURIFICATION DU MONTÉLUKAST

(30) Priority: 09.08.2006 EP 06118689; 09.08.2006 US 836873 P
(43) Date of publication of application: 10.06.2009
(73) Proprietor: ESTEVE QUIMICA, S.A., 08024 Barcelona (ES)
(72) Inventor: GASANZ GUILLEN, Yolanda, E-08024 Barcelona (ES); MONSALVATJE LLAGOSTERA, Montserrat, E-08024 Barcelona (ES); TALAVERA ESCASANY, Pedro, E-08024 Barcelona (ES)
(74) Representative: ZBM Patents
(86) International application number: PCT/EP2007/058176
(87) International publication number: WO 2008/017667

(56) References cited:
- WO-A-2006/058545
- WO-A2-2006/064269

## Description

The present invention relates to a process for the preparation of highly pure Montelukast. In particular, it relates to a purification process of Montelukast by selective extractions of Montelukast or its impurities in a mixture of an organic solvent and water.

### BACKGROUND ART

Montelukast, is the International Non-proprietary Name (INN) of 1-[[[(1 R)-1-[3-[(1E)-2-(7-chloro-2-quinolinyl)ethenyl]phenyl]-3-[2-(1-hydroxy-1-methylethyl)phenyl]propyl]sulfanyl]methyl]cyclopropaneacetic acid, and CAS No. 158966-92-8. Montelukast sodium salt (CAS No 151767-02-1) is currently used in treatment of asthma, inflammation, angina, cerebral spasm, glomerular nephritis, hepatitis, endotoxemia, uveitis and allograft rejection.

The structure of Montelukast sodium salt corresponds to formula (I):

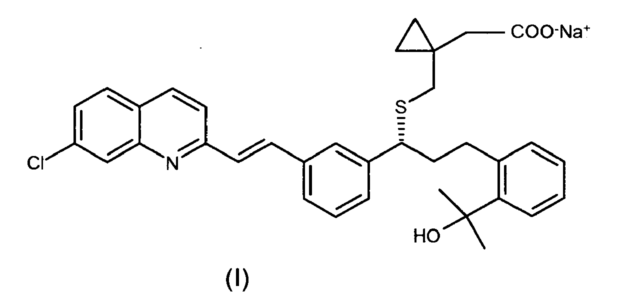

Different synthetic strategies for the preparation of Montelukast and its salts are known. For instance, EP 480.717 discloses certain substituted quinolone compounds including Montelukast sodium salt, methods for their preparation, and pharmaceutical compositions using these compounds. Several preparation processes of Montelukast sodium salt are reported in this document. Example 161 relates to the preparation of Montelukast sodium salt. According to this Example, preparation of Montelukast sodium salt proceeds through its corresponding methyl ester, whose preparation comprises sodium hydride or cesium carbonate assisted coupling of methyl-1-(mercaptomethyl)-cyclopropaneacetate with the protected mesylate (2-(2-(2-(3(S)-(3-(2-(7-chloro-2-quinolinyl)-ethenyl)phenyl)-3-(methanesulfonyloxy)propyl)phenyl)-2-propoxy)tetrahydropyran, generated in situ. The methyl ester thus obtained is hydrolyzed to the Montelukast acid which is then converted directly to the sodium salt. This process is not particularly suitable for large scale production because it requires tedious chromatographic purification of the methyl ester intermediate and/or the final product, and yields of intermediates and final product are low. Other methods for the preparation of Montelukast and its salts have been described (cf. e.g. WO 04/108679, US 2005/107612, WO 05/105751, WO 05/105749, WO 05/105750, CN 1428335, and CN 1420113).

Generally, Montelukast and its pharmaceutically acceptable salts are obtained by complex synthetic procedures which cause the formation of several by-products due to competing side reactions. These processes need tedious workups to isolate the Montelukast and its intermediates and thus results in excess time cycle, which in turn rendering the process more costly and less eco friendly. It is known that the purification of Montelukast is laborious and complex, being difficult to achieve a Montelukast with a high degree of purity since Montelukast and its precursors are unstable to oxygen and light causing a fast degradation. For the above reasons, Montelukast is generally obtained with a low degree of chemical and optical purity.

Some processes for the purification of Montelukast have been described in the art which are based on the formation of its salts. Thus, EP 737.186 relates to a process for the preparation of Montelukast or its salts thereof, which comprises reacting the dilithium dianion of 1-(mercaptomethyl)-cyclopropane-acetic acid with the corresponding mesylate alcohol ((2-(2-(2-(3(S)-(3-(2-(7-chloro-2-quinolinyl)-ethenyl)phenyl)-3-(methanesulfonyloxy)-propyl)phenyl)-2-propanol), to obtain Montelukast. The crude acid is purified through the formation of its dicyclohexylamine salt. Depending on the solvent used two crystalline forms of the salts can be obtained, so seeding play a very critical role during crystallization. Patent application US 2005/234241 also describes a process for the preparation of Montelukast which occurs via the formation of organic Montelukast base salts. In particular, example 2 describes the formation of the tert-butylamine salt of Montelukast. Patent application WO 06/008751 also describes a process for the preparation of Montelukast and a process for its purification via the formation of several organic Montelukast base salts.

According to WO 05/074935, Montelukast sodium can be purified by obtaining montelukast free acid as a solid and converting the montelukast free acid into montelukast sodium.

Although certain processes for the purification of Montelukast or its salts are known, there is a need for a purification process that provides a Montelukast in high yield and with a high purity. Thus, the provision of a new easily industrializable process for the purification of Montelukast and its salts, which achieve the elimination or at least the drastic reduction, below the corresponding limit of acceptability, of the impurities of Montelukast, would be of great interest in industry.

### SUMMARY OF THE INVENTION

Inventors have found a new efficient purification process of Montelukast which proceeds with high yield and which allows to obtain Montelukast with high chemical purity. The process is based on carrying out a specific set of solvent extractions at specific ranges of pH and temperature which depends on the type of impurities to remove. Montelukast can be purified by an easily industrializable process, which is simple and cost-effective, does not use toxic solvents and do not involve significant waste of product.

Thus, according to an aspect of the present invention it is provided a process for the purification of Montelukast, or pharmaceutically salts thereof, or solvates thereof, including stereoisomers or mixtures thereof, which comprises carrying out a specific set of selective extractions of Montelukast or its impurities, in a mixture of an organic solvent and water, at specific ranges of pH and temperature, said set of solvent extractions comprising at least one wash of an aqueous phase containing crude Montelukast in salt form with an organic solvent, at a pH comprised between 12.0 and 13.5 and at a temperature comprised between 10°C and about 5 °C below the boiling point of the mixture. With the process of the present invention specific impurities derived of the synthetic routes used to prepare Montelukast are easily purified.

The process of the present invention is especially useful when Montelukast is obtained by hydrolysis of the corresponding cyano intermediate of formula (II).

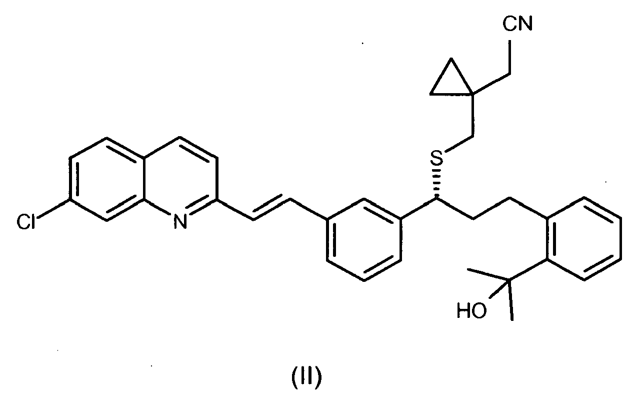

In such a case the process of the present invention allows to purify some specific impurities derived of this process which are very difficult to purify by other known methods.

### DETAILED DESCRIPTION OF THE INVENTION

The purification process of the present invention can be used to purify Montelukast prepared according to any known process of the prior art. As it is said above, the process for the purification of Montelukast of formula (I), or its pharmaceutically acceptable salts, or its solvates, including stereoisomers or mixtures thereof, comprises carrying out a specific set of selective extractions of Montelukast or its impurities, in a mixture of an organic solvent and water. Among the impurities that may be effectively removed with the purification process of the present invention are the following ones.

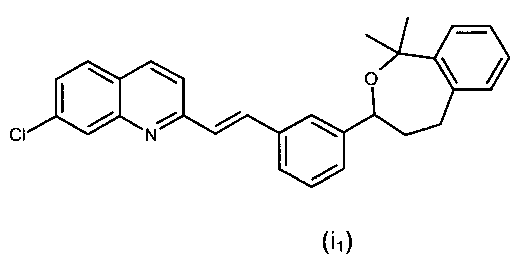

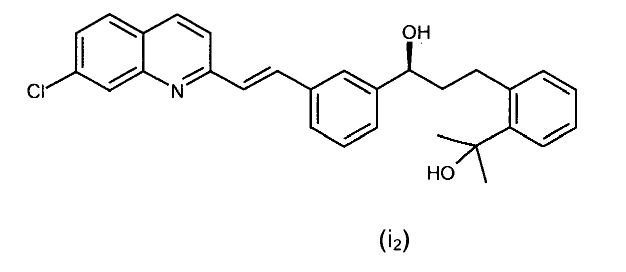

These impurities generally present in Montelukast are effectively removed with solvent extractions of an aqueous phase containing Montelukast in salt form, at a pH comprised between 12.0 and 13.5.

Other impurities that may be present in Montelukast are the following ones.

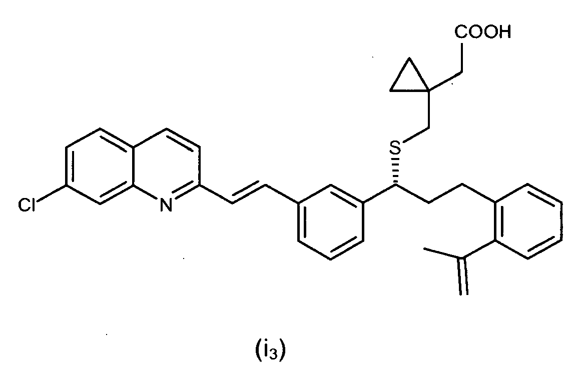

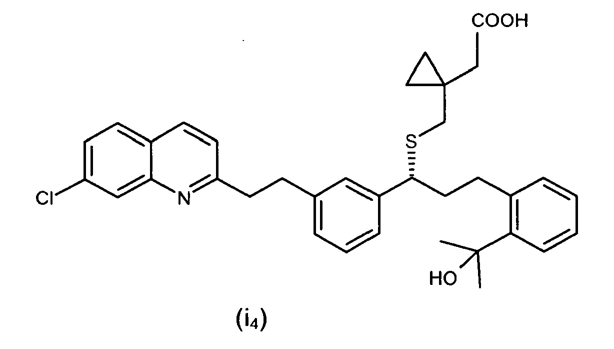

These impurities, if they are present, may be effectively removed with solvent extractions of an aqueous phase containing Montelukast in salt form at a pH comprised between 8.5 and 10.0.

The purification process is especially useful to purify Montelukast obtained from the corresponding cyano intermediate of formula (II) mentioned above. The specific impurities derived of this process may be effectively removed with the solvent extractions of the aqueous phase containing Montelukast in salt form, at a pH comprised between 12.0 and 13.5. Among these impurities are the cyano compound used as starting material and the following impurities:

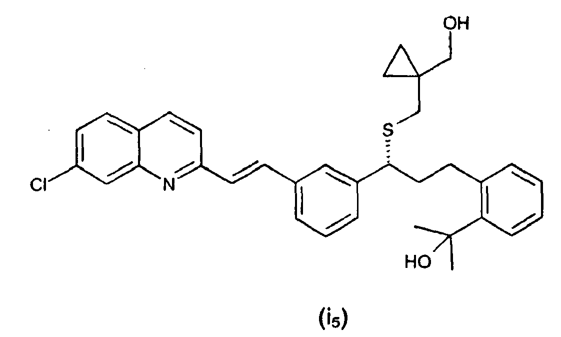

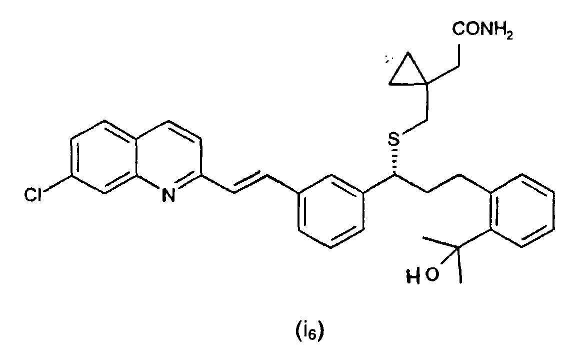

Thus, in a particular embodiment, the purification process further comprises a previous step where a compound of formula (II), 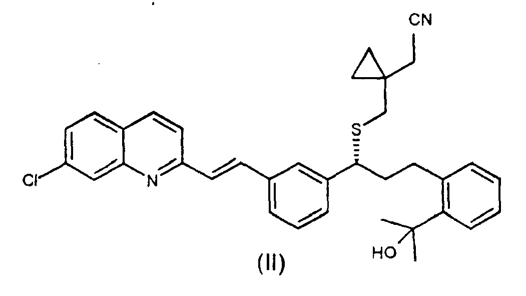 is reacted with an inorganic base in a mixture of an (C₁-C₆)-alcohol and water, to give Montelukast crude of formula (I) in form of salt. In such a case, at the end of the reaction, the alcohol may be separated, for instance, by distillation. Then an organic solvent and water can be added to the crude of the reaction in order to separate an aqueous phase containing the salts from the organic phase containing the Montelukast in salt form. The Montelukast in salt form can be extracted of the organic phase with water. To the resulting aqueous solution containing the Montelukast in salt form is carried out the purification process of the present invention.

The purification process of the present invention can also be carried out from an aqueous solution containing the Montelukast in salt form previously obtained by adding to a mixture of Montelukast acid in an organic solvent, an aqueous solution of a base.

In a particular embodiment, the purification process comprises the following steps: (a) carrying out at least one wash of an aqueous phase containing crude Montelukast in salt form with an organic solvent, at a pH comprised between 12.0 and 13.5 and at a temperature comprised between 10°C and about 5 °C below the boiling point of the mixture and separating the aqueous phase containing the Montelukast in salt form; (b) optionally, carrying out one or more washes of the aqueous phase of step (a) with an organic solvent at a pH comprised between 8.5 and 10.0 and at a temperature comprised between 10°C and about 5 °C below the boiling point of the mixture, and separating the aqueous phase containing the Montelukast in salt form; (c) carrying out an extraction of the purified Montelukast from the aqueous phase of steps (a) or (b) with an organic solvent at a pH comprised between 4.5 and 8.0 and at a temperature comprised between 10°C and about 5 °C below the boiling point of the mixture, and separating the organic phase containing the Montelukast acid; and (d) optionally, isolating the Montelukast from the organic phase of step (c) as acid or in salt form.

Preferably, the organic solvent used in the purification process is selected from the group consisting of (C₂-C₈)- ether, (C₆-C₈)-aromatic hydrocarbon, (C₁-C₃)-chlorine containing solvents and mixtures thereof. More preferably, the solvent is selected from toluene, tert-butyl methyl ether, tetrahydrofuran, and mixtures thereof.

Preferably, two or three washes at a pH comprised between 12.0 and 13.5 are carried out. Preferably, said washes of step (a) are carried out at a pH comprised between 12.0 and 13.5 and at a temperature comprised between 20 and 60°C. More, preferably, at least two washes are carried out at set pH and temperature. Also preferably, the washes of step (b) are carried out at a pH comprised between 9.0 and 9.5 and at a temperature comprised between 20 and 60°C. The most appropriate temperature conditions vary depending mainly on the solvent used. The temperature can be readily determined by the skilled person in the art with the help of the teaching of the examples given in the description.

In a preferred embodiment, the Montelukast is isolated from the organic phase of step (c) as acid, for instance, by filtration, and optionally, is treated with a base in the presence of an appropriate solvent to form the corresponding pharmaceutically acceptable salt of Montelukast, which is isolated from the reaction medium, for instance, by filtration.

In another preferred embodiment the Montelukast is isolated from the organic phase of step (c) in salt form by adding a base and isolating from the reaction medium the corresponding pharmaceutically acceptable salt of Montelukast, for instance, by filtration.

The preparation of purified Montelukast by the process of the present invention involves an important improvement over the known processes. Among the advantageous features of the purification process of the present invention, the following can be mentioned: (i) it allows to purify Montelukast from several impurities that are difficult to purify by known methods; (ii) it is able to purify Montelukast crude with a low degree of purity and achieving a high yield; and (iii) the Montelukast is manipulated during all the process in solution up to its isolation in solid form as acid or as a salt, so that repeatedly recrystallizations of Montelukast are avoided.

Throughout the description and claims the word "comprise" and variations of the word, are not intended to exclude other technical features, additives, components, or steps. The abstract of this application is incorporated herein as reference. Additional objects, advantages and features of the invention will become apparent to those skilled in the art upon examination of the description or may be learned by practice of the invention. The following examples are provided by way of illustration, and they are not intended to be limiting of the present invention.

### EXAMPLES

### Example 1: Preparation of Montelukast acid

143.6 g of sodium hydroxide were added to a solution of 102 g of (R,E)-2-(1-((1-(3-(2-(7-chloroquinolin-2-yl)vinyl)phenyl)-3-(2-(2-hydroxypropan-2-yl)phenyl)propylthio)methyl)cyclopropyl)acetonitrile in 407 ml of ethanol 96% (v/v). The mixture was stirred at reflux temperature for 30 hours. After this period of time, the solvent was distilled off under vacuum (Purity by HPLC: 61.5 area %; impurity i₂: 1.84 area %; impurity i₅: 3.70 area %; impurity i₆ : 1.42 area %). The mixture was partitioned with 1000 ml of toluene and 1500 ml of water at room temperature. The aqueous phase dissolved the inorganic salts was discarded. The organic phase was mixed with 1500 ml of water and the mixture was heated to 60 °C and stirred for 15 minutes. At this point the product was dissolved in the aqueous layer. The pH of the aqueous layer was 12.5. Some impurities from the process were dissolved in the organic layer. Therefore, the organic layer was discarded.The toluene wash at 60°C was repeated. The outcome aqueous layer was cooled down to room temperature and 1000 ml of toluene were added. Then, the pH of the mixture was adjusted to 5.6 with a 2M solution of acetic acid. The mixture was stirred for 30 minutes and the aqueous layer was discarded. Finally, the organic layer was washed with 1000 ml of water and kept as a solution of purified Montelukast acid (Purity by HPLC: 84.4 area %; impurity i₂ : 0.81 area %; impurity i₅: 0.70 area %; impurity i₆: 0.07 area %). Yield of the title compound from the outcome solution: 86%.

### Example 2: Crystallization of Montelukast acid

The toluene solution obtained in the previous example was stirred at room temperature for 12 hours. After this period of time, a yellow suspension was formed. The outcome solid was filtered off, washed with toluene and dried under vacuum at 30 °C for 24 hours. 53 g of Montelukast acid were recovered (Purity by HPLC: 96 area %). Yield: 90%.

### Example 3: Purification of Montelukast acid using toluene as solvent for the extractions

45 ml of an aqueous solution of NaOH 0.5M were added to a 30 ml suspension of Montelukast acid (impurity i₂: 0.05 area %; impurity i₃: 1.39 area %; impurity i₅ : 0.20 area %; impurity i₆: 0.05 area %) and toluene. A two layer solution was formed where Montelukast appeared dissolved in the aqueous layer as a sodium salt. After 30 minutes of stirring at 40 °C, the organic layer was discarded. Another two more washes with 30 ml of toluene were successively carried out adjusting the pH each time between 12.2 and 13.2. The outcome aqueous solution was acidified to pH 9.3 with an aqueous 2M solution of acetic acid and washed twice with 30 ml of toluene. Both extractions were carried out at 60 °C. Finally, another 30 ml of toluene were added to the aqueous solution and the mixture was acidified to pH 6.0 with an aqueous 2M solution of acetic acid at room temperature. The final organic layer was separated and kept as a solution of purified Montelukast acid.
(Purity by HPLC: 96.8 area %; impurity i₂: 0.02 area %; impurity i₃: 1.23 area %; impurity i₅ : not detected; impurity i₆ : not detected %). Yield: 83%.

### Example 4: Purification of Montelukast acid using tert-butyl mehtyl ether as solvent for the extractions

45 ml of an aqueous solution of NaOH 0.5M were added to a 30 ml suspension of Montelukast acid (Purity by HPLC: 97.4 area %; impurity i₂ : 0.05 area %; impurity i₃ : 1.39 area %; impurity i₅ : 0.20 area %; impurity i₆ : 0.05 area %) and tert-butyl methyl ether. A two layer solution was formed where Montelukast appeared dissolved in the aqueous layer as a sodium salt. After 30 minutes of stirring at room temperature, the organic layer was discarded. Another two more washes with 30 ml of tert-butyl methyl ether were successively carried out adjusting the pH each time between 12.5 and 13.5 at room temperature. The outcome aqueous solution was acidified to pH 9.2 with an aqueous 2M solution of acetic acid and washed twice with 30 ml of tert-butyl methyl ether at room temperature.. Finally, another 30 ml of toluene were added to the aqueous solution and the mixture was acidified to pH 7.7 with an aqueous 2M solution of acetic acid. The final organic layer was separated and kept as a solution of purified Montelukast acid (Purity by HPLC: 97.7 area %; impurity i₂ :not detected; impurity i₃ : 1.11 area %; impurity i₅ : not detected; impurity i₆ : not detected). Yield: 67%.

### Example 5: Preparation of Montelukast sodium

2.6 g of Montelukast acid were dissolved in 26 ml of toluene and 8.9 ml of 0.5M NaOH solution in methanol were added slowly at room temperature. The mixture was stirred for 1 hour and the solvent was removed under vacuum to obtain a residue. Then, heptane (24 ml) was added over 30 minutes to a well stirred solution of the residue in 4 ml of ethyl acetate at room temperature. Two hours after the addition, an off white solid was filtered off under a nitrogen atmosphere and washed with 5 ml of heptane. The wet product was dried under vacuum at 70-80 °C for 2 days to yield 2.7 g of Montelukast sodium (purity by HPLC: 99 area %). Yield: 100%.

### Example 6: Preparation of Montelukast sodium

34.1 ml of 0.5M NaOH solution in methanol were added slowly at room temperature to 114.3 ml of the solution obtained in the Example 1. The mixture was stirred for 1 hour and the solvent was removed under vacuum to obtain a residue. Then, 90 ml of heptane were added over 30 minutes to a well stirred solution of the residue in 15 ml of ethyl acetate at room temperature. Two hours after the addition, an off white solid was filtered off under a nitrogen atmosphere and washed with 20 ml of heptane. The wet product was dried under vacuum at 70-80 °C for 2 days to yield 7.6 g of Montelukast sodium. Yield: 73%.

## Claims

1. A process for the purification of Montelukast, or a pharmaceutically salt thereof, or a solvate thereof, including any stereoisomer or mixture thereof which comprises carrying out a specific set of selective solvent extractions of Montelukast or its impurities, said set of solvent extractions comprising at least one wash of an aqueous phase containing crude Montelukast in salt form with an organic solvent, at a pH comprised between 12.0 and 13.5 and at a temperature comprised between 10 °C and about 5 °C below the boiling point of the mixture.

2. The purification process according to claim 1, which comprises the following steps:
(a) carrying out at least one wash of an aqueous phase containing crude Montelukast in salt form with an organic solvent, at a pH comprised between 12.0 and 13.5 and at a temperature comprised between 10 °C and about 5 °C below the boiling point of the mixture, and separating the aqueous phase containing the Montelukast in salt form;
(b) optionally, carrying out one or more washes of the aqueous phase of step (a) with an organic solvent at a pH comprised between 8.5 and 10.0 and at a temperature comprised between 10 °C and about 5 °C below the boiling point of the mixture, and separating the aqueous phase containing the Montelukast in salt form;
(c) carrying out an extraction of the purified Montelukast from the aqueous phase of steps (a) or (b) with an organic solvent at a pH comprised between 4.5 and 8.0 and at a temperature comprised between 10 °C and about 5 °C below the boiling point of the mixture, and separating the organic phase containing the Montelukast acid; and
(d) optionally, isolating the Montelukast from the organic phase of step (c) as acid or in salt form.

3. The purification process according to any of the claims 1-2, wherein the organic solvent is selected from the group consisting of (C₂-C₈)- ether, (C₆-C₈)-aromatic hydrocarbon; (C₁-C₃)-chlorine containing solvents, and mixtures thereof.

4. The purification process according to claim 3, wherein the solvent is selected from toluene, tert-butyl methyl ether, tetrahydrofuran, and mixtures thereof.

5. The purification process according to any of the claims 1-4, wherein at least are carried out two washes at a pH equal to 12.0-13.5 and at a temperature comprised between 20 and 60°C.

6. The purification process according to any of the claims 1-5 wherein the washes of step (b) are carried out at a pH comprised between 9.0 and 9.5, and at a temperature comprised between 20 and 60°C.

7. The purification process according to any of the claims 1-6, wherein the Montelukast is isolated from the organic phase of step (c) as acid, and optionally, is treated with a base to form a pharmaceutically acceptable salt of Montelukast.

8. The purification process according to claim 7, wherein the Montelukast acid is isolated by filtration.

9. The purification process according to any of the claims 1-6, wherein the Montelukast is isolated from the organic phase of step (c) in salt form by adding a base and isolating from the reaction medium the corresponding pharmaceutically acceptable salt of Montelukast.

10. The purification process according to claim 9, wherein the salt of Montelukast is isolated by filtration.

11. The purification process according to any of the claims 9-10, wherein the base is sodium hydroxide.

12. The purification process according to any of the claims 1-11, further comprising a previous step where a compound of formula (II) 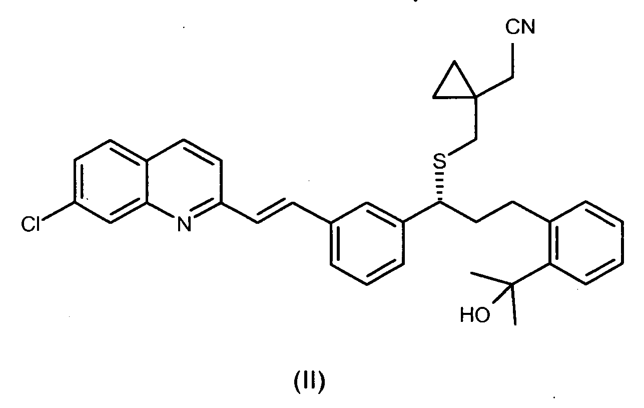 is reacted with an inorganic base in a mixture of an (C₁-C₆)-alcohol and water, to yield Montelukast crude.

13. The purification process according to claim 12, further comprising:
(a) optionally, separating the alcohol from the reaction medium;
(b) adding an organic solvent and water and separating the aqueous phase; and
(c) carrying out an extraction of the Montelukast in salt form from the organic phase with water; to yield an aqueous phase containing Montelukast crude in salt form.

14. The purification process according to any of the claims 1-11, wherein the aqueous phase containing crude Montelukast in salt form is obtained by adding an aqueous solution of a base to a mixture of Montelukast acid and an organic solvent.

## Patentansprüche

1. Verfahren zur Aufreinigung von Montelukast, oder eines pharmazeutisch verträglichen Salzes davon, oder eines Solvats davon, einschließlich jeglichen Stereoisomers oder jeglicher Mischung davon, bei dem eine bestimmte Reihe selektiver Solventextraktionen von Montelukast oder dessen Verunreinigungen ausgeführt wird, wobei die besagte Reihe von Solventextraktionen mindestens einen Waschdurchgang einer wässrigen Phase, die Rohmontelukast in Salzform enthält, mit einem organischen Lösungsmittel bei einem pH-Wert zwischen 12,0 und 13,5 und bei einer Temperatur zwischen 10 °C und zirka 5 °C unter dem Siedepunkt der Mischung umfasst.

2. Aufreinigungsverfahren nach Anspruch 1 mit den folgenden Verfahrensschritten:
(a) mindestens einmaliges Waschen einer wässrigen Phase, die Rohmontelukast in Salzform enthält, mit einem organischen Lösungsmittel bei einem pH-Wert zwischen 12,0 und 13,5 und bei einer Temperatur zwischen 10 °C und zirka 5 °C unter dem Siedepunkt der Mischung, und Abtrennen der wässrigen Phase, die das Montelukast in Salzform enthält;
(b) wahlweise ein- oder mehrmaliges Waschen der wässrigen Phase aus Verfahrensschritt (a) mit einem organischen Lösungsmittel bei einem pH-Wert zwischen 8,5 und 10,0 und bei einer Temperatur zwischen 10 °C und zirka 5 °C unter dem Siedepunkt der Mischung, und Abtrennen der wässrigen Phase, die das Montelukast in Salzform enthält;
(c) Extrahieren des aufgereinigten Montelukasts aus der wässrigen Phase aus Verfahrensschritt (a) oder (b) mit einem organischen Lösungsmittel bei einem pH-Wert zwischen 4,5 und 8,0 und bei einer Temperatur zwischen 10 °C und zirka 5 °C unter dem Siedepunkt der Mischung, und Abtrennen der organischen Phase, die die Montelukastsäure enthält; und
(d) wahlweise Isolieren des Montelukasts aus der organischen Phase aus Verfahrensschritt (c) als Säure oder in Salzform.

3. Aufreinigungsverfahren nach einem der Ansprüche 1-2, bei dem das organische Lösungsmittel aus der Gruppe ausgewählt wird, die aus (C₂-C₈)-Ether, (C₆-C₈)-aromatischem Kohlenwasserstoff; (C₁-C₃)-chlorhaltigem Lösungsmittel und Mischungen daraus besteht.

4. Aufreinigungsverfahren nach Anspruch 3, bei dem das Lösungsmittel aus Toluol, tert-Butylmethylether, Tetrahydrofuran, und Mischungen daraus ausgewählt wird.

5. Aufreinigungsverfahren nach einem der Ansprüche 1-4 mit mindestens zweimaligem Waschen bei einem pH-Wert, der 12,0 bis 13,5 beträgt, und bei einer Temperatur zwischen 20 und 60 °C.

6. Aufreinigungsverfahren nach einem der Ansprüche 1-5, bei dem die Waschdurchgänge des Verfahrensschrittes (b) bei einem pH-Wert zwischen 9,0 und 9,5 und bei einer Temperatur zwischen 20 und 60 °C ausgeführt werden.

7. Aufreinigungsverfahren nach einem der Ansprüche 1-6, bei dem das Montelukast als Säure von der organischen Phase aus Verfahrensschritt (c) isoliert wird, und wahlweise mit einer Base behandelt wird, damit ein pharmazeutisch verträgliches Salz von Montelukast gebildet wird.

8. Aufreinigungsverfahren nach Anspruch 7, bei dem die Montelukastsäure durch Filtrieren isoliert wird.

9. Aufreinigungsverfahren nach einem der Ansprüche 1-6, bei dem das Montelukast als Salz von der organischen Phase aus Verfahrensschritt (c) isoliert wird, indem man eine Base zugibt und das sich ergebende pharmazeutisch verträgliche Montelukastsalz aus dem Reaktionsmedium isoliert.

10. Aufreinigungsverfahren nach Anspruch 9, bei dem das Montelukastsalz durch Filtrieren isoliert wird.

11. Aufreinigungsverfahren nach einem der Ansprüche 9-10, bei dem die Base Natriumhydroxid ist.

12. Aufreinigungsverfahren nach einem der Ansprüche 1-11, bei dem weiterhin in einem vorgeschalteten Verfahrensschritt eine Verbindung der Formel (II) 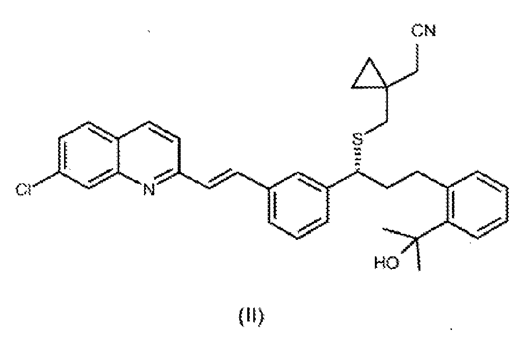 mit einer anorganischen Base in einer Mischung aus (C₁-C₆)-Alkohol und Wasser zur Reaktion gebracht wird, um Rohmontelukast zu ergeben.

13. Aufreinigungsverfahren nach Anspruch 12, bei dem weiterhin:
(a) wahlweise der Alkohol aus dem Reaktionsmedium entfernt wird;
(b) ein organisches Lösungsmittel und Wasser hinzugefügt werden und die wässrige Phase abgetrennt wird; und
(c) das Montelukast in Salzform mit Wasser aus der organischen Phase extrahiert wird, um eine wässrige Phase, die Rohmontekulast in Salzform enthält, zu ergeben.

14. Aufreinigungsverfahren nach einem der Ansprüche 1-11, bei dem die wässrige Phase, die Rohmontelukast in Salzform enthält, gewonnen wird, indem man einer Mischung aus Montelukastsäure und einem organischen Lösungsmittel eine wässrige Lösung einer Base zugibt.

## Revendications

1. Procédé pour la purification de Montélukast, ou d'un sel acceptable sur le plan pharmaceutique de celui-ci, ou d'un solvate de celui-ci, comprenant n'importe quel stéréo-isomère ou mélange de ceux-ci lequel procédé comprend la réalisation d'un ensemble spécifique d'extractions sélectives par solvant de Montélukast ou de ses impuretés, ledit ensemble d'extraction par solvant comprenant au moins un lavage d'une phase aqueuse contenant du Montélukast brut sous forme de sel avec un solvant organique, à un pH compris entre 12,0 et 13,5 et à une température comprise entre 10 °C et environ 5 °C en dessous du point d'ébullition du mélange.

2. Procédé de purification selon la revendication 1, qui comprend les étapes suivantes :
(a) la réalisation d'au moins un lavage d'une phase aqueuse contenant du Montélukast brut sous forme de sel avec un solvant organique, à un pH compris entre 12,0 et 13,5 et à une température comprise entre 10°C et environ 5 °C en dessous du point d'ébullition du mélange, et la séparation de la phase aqueuse contenant le Montélukast sous forme de sel ;
(b) éventuellement, la réalisation d'un ou plusieurs lavages de la phase aqueuse de l'étape (a) avec un solvant organique, à un pH compris entre 8,5 et 10,0 et à une température comprise entre 10 °C et environ 5 °C en dessous du point d'ébullition du mélange, et la séparation de la phase aqueuse contenant le Montélukast sous forme de sel ;
(c) la réalisation d'une extraction du Montélukast purifié provenant de la phase aqueuse des étapes (a) ou (b) avec un solvant organique, à un pH compris entre 4,5 et 8,0 et à une température comprise entre 10 °C et environ 5 °C en dessous du point d'ébullition du mélange, et la séparation de la phase organique contenant l'acide de Montélukast ; et
(d) éventuellement, l'isolement du Montélukast de la phase organique de l'étape (c) en tant qu'acide ou sous forme de sel.

3. Procédé de purification selon l'une quelconque des revendications 1 à 2, dans lequel le solvant organique est choisi dans le groupe constitué d'un éther en (C₂ à C₈), un hydrocarbure aromatique en (C₆ à C₈) des solvants chlorés en (C₁ à C₃), et leurs mélanges.

4. Procédé de purification selon la revendication 3, dans lequel le solvant est choisi parmi le toluène, le tert-butyl méthyl-éther, le tétrahydrofurane, et leurs mélanges.

5. Procédé de purification selon l'une quelconque des revendications 1 à 4, dans lequel on réalise au moins deux lavages à un pH égal à 12,0 à 13,5 et à une température comprise entre 20 et 60 °C.

6. Procédé de purification selon l'une quelconque des revendications 1 à 5, dans lequel les lavages de l'étape (b) sont effectués à un pH compris entre 9,0 et 9,5, et à une température comprise entre 20 et 60 °C.

7. Procédé de purification selon l'une quelconque des revendications 1 à 6, dans lequel le Montélukast est isolé de la phase organique de l'étape (c) en tant qu'acide, et éventuellement, est traité avec une base pour former un sel acceptable sur le plan pharmaceutique de Montélukast.

8. Procédé de purification selon la revendication 7, dans lequel l'acide de Montélukast est isolé par filtration.

9. Procédé de purification selon l'une quelconque des revendications 1 à 6, dans lequel le Montélukast est isolé de la phase organique de l'étape (c) sous forme de sel en ajoutant une base et en isolant du milieu réactionnel le sel acceptable sur le plan pharmaceutique correspondant de Montélukast.

10. Procédé de purification selon la revendication 9, dans lequel le sel de Montélukast est isolé par filtration.

11. Procédé de purification selon l'une quelconque des revendications 9 à 10, dans lequel la base est l'hydroxyde de sodium.

12. Procédé de purification selon l'une quelconque des revendications 1 à 11, comprenant en outre une étape précédente où un composé de formule (II) 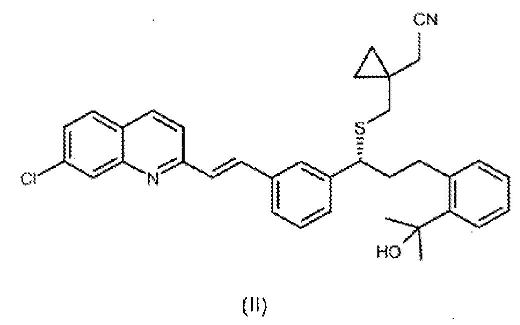 est mis en réaction avec une base inorganique dans un mélange d'un alcool en (C₁ à C₆) et d'eau, pour donner un produit brut de Montélukast.

13. Procédé de purification selon la revendication 12, comprenant en outre :
(a) éventuellement, la séparation de l'alcool du milieu réactionnel ;
(b) l'ajout d'un solvant organique et d'eau et la séparation de la phase aqueuse ; et
(c) la réalisation d'une extraction du Montélukast sous forme de sel de la phase organique avec de l'eau ; pour donner une phase aqueuse contenant du produit brut de Montélukast sous forme de sel.

14. Procédé de purification selon l'une quelconque des revendications 1 à 11, dans lequel la phase aqueuse contenant le Montélukast brut sous forme de sel est obtenue en ajoutant une solution aqueuse d'une base à un mélange d'acide de Montélukast et d'un solvant organique.
